# EUROPÄISCHE PATENTSCHRIFT

(11) **EP 0 864 329 B1**
(45) Veröffentlichungstag und Bekanntmachung des Hinweises auf die Patenterteilung: **13.12.2000**
(21) Anmeldenummer: 97113757.5
(22) Anmeldetag: 08.08.1997
(51) Int. Cl.: A61K 33/00, A61K 47/02, A61K 9/00, A61K 9/08

(54) **Medizinische Präparation, die ein lipophiles Edelgas enthält**
Medical preparations containing a lipophile noble gas
Préparations médicales contenant un gaz noble lipophile

(30) Priorität: 10.03.1997 DE 19709704
(43) Veröffentlichungstag der Anmeldung: 16.09.1998
(73) Patentinhaber: Georgieff, Michael, 89081 Ulm (DE)
(72) Erfinder: Georgieff, Michael, 89081 Ulm (DE)
(74) Vertreter: Wibbelmann, Jobst, Dr., Dipl.-Chem.

(56) Entgegenhaltungen:
- EP-A- 0 523 315
- WO-A-85/00011
- WO-A-95/27438
- DE-A- 1 667 926
- DE-A- 3 940 389
- DE-A- 4 100 782

## Beschreibung

Die Erfindung betrifft eine flüssige Präparation, die ein lipophiles Edelgas in einer pharmakologisch wirksamen Konzentration enthält.

Lipophile Edelgase sind solche Edelgase, die eine gewisse Fettlöslichkeit haben. Ausdruck hierfür ist beispielsweise ein Öl-Gas-Koeffizient größer als etwa 0,05 (Krypton, 0,5; Argon, 0,15; Xenon, 1,9).

Unter pharmakologisch oder pharmazeutisch wirksam wird hier eine Konzentration in der flüssigen Präparation verstanden, die imstande ist, bei einem Patienten sedierend, anästhesierend, analgetisch, antiinflammatorisch oder muskelrelaxierend zu wirken.

Xenon wird unter anderem als Inhalationsnarkotikum diskutiert, da dieses Edelgas eine narkotisierende und schmerzstillende Wirkung hat. Da Xenon sehr teuer, die Anwendung als Inhalationsanästhetikum mit einem hohen Verbrauch einhergeht und zudem die Behandlung mit einem Gas technisch sehr aufwendig ist, hat die Anästhesie mit Xenon keine Verbreitung gefunden. Allerdings gibt es wegen der offensichtlichen Vorteile von Xenongas gegenüber anderen gasförmigen Narkosemitteln Bestrebungen, entweder über eine einfachere und preisgünstigere Gewinnung des Xenons oder Rückgewinnung des Xenons aus der ausgeatmeten Luft den Einsatz dieses Gases in großem Umfang zu ermöglichen.

Bei Xenon handelt es sich um ein einatomiges farb-, geruch- und geschmackloses Edelgas der Ordnungszahl 54. Xenon ist fünfmal schwerer als Luft. Natürliches Xenon enthält des weiteren Isotope, beispielsweise die Isotope 124, 126, 128, 129, 130, 131, 132, 134 und 136. Daneben sind auch künstliche Isotope bekannt sowie Xenon 114, Xenon 133 und Xenon 142. Diese Isotope zerfallen mit Halbwertszeiten, die zwischen 1,2 Sekunden und etwa 40 Tagen liegen. Radioaktive Xenon-Isotope werden von der vorliegenden Erfindung nicht angesprochen.

Bei der Anwendung von Xenon als Inhalationsanästhetikum sind einerseits sehr große Mengen erforderlich, um eine narkotische Wirkung zu erreichen, andererseits ist man in der inspiratorischen Konzentration auf 70 %, maximal 79 % limitiert, um die Versorgung eines Patienten mit mindestens 21 % Sauerstoff in der Inspirationsluft zu gewährleisten. Dabei wird eine gewisse Anästhesie und Analgesie ermöglicht, die für sich alleine genommen aber nicht ausreicht, um eine adäquate allgemeine Anästhesie eines Patienten zu gewährleisten. Es müssen deshalb zusätzliche Beruhigungsmittel (Sedativa) oder intravenöse Anästhetika sowie Analgetika supplementiert, d.h. ergänzt werden. Bei intraabdominellen oder intrathorakalen Eingriffen müssen ebenfalls Muskelrelaxanzien ergänzt werden.

Es ist nicht bekannt, daß jemals der Versuch unternommen wurde, eine flüssige Präparation, die lipophile Edelgase enthält, als Injektionsnarkosemittel einzusetzen. Es ist weiterhin nicht bekannt, solche Präparationen auch zu anderen medizinischen Zwecken wie beispielsweise der Analgesie oder Sedierung einzusetzen.

Die DE-A-39 40 389 beschreibt ein therapeutisches Mittel, das ein Gas in einer über deren natürlichen Gas-Sättigungsgrad liegenden Konzentration angereichert enthält. Als Gas wird unter anderem Luftsauerstoff, Ozon oder ein Edelgas erwähnt. Ausführlich erläutert diese Druckschrift, daß das therapeutische Mittel für Zwecke der Katastrophenmedizin und der Schockbekämpfung einzusetzen ist, insbesondere dann, wenn einem Patienten durch Infusion dieses Mittel als Blutersatzmittel und als Sauerstofftransporter verabreicht wird. Als erfindungsgemäßes Mittel wird insbesondere eine isotonische Kochsalzlösung bezeichnet, die bis zu 40 mg/l Sauerstoff enthält. Über die mögliche Wirksamkeit von Edelgasen oder Einsatzgebiete eines Mittels, das Edelgase enthält, macht die Druckschrift keinerlei Angaben.

Aus der DE-A-16 67 926 ist eine pharmakologisch unbedenkliche Salzlösung bekannt, die ein radioaktives Gas enthält. Radioaktive Gase werden von der vorliegenden Erfindung nicht angesprochen.

Die DE-C-41 00 782 beschreibt wäßrige Ozonpräparate, die als Infusionslösung einem Patienten zugeführt werden können. Diese Druckschrift hebt aber hervor, daß Ozon bestimmte algizide, bakterizide, fungizide, sporizide und viruzide Wirkungen hat. Darüberhinaus wird angesprochen, daß Ozon in Sekundenbruchteilen mit den ungesättigten Fettsäuren des Blutes reagiert. Wegen des raschen Zerfalls des Ozons wird empfohlen, daß die Infusionslösungen am Ort des Verbrauchs hergestellt werden.

Neben den Inhalationsnarkotika werden im Stand der Technik Injektionsnarkotika beschrieben. Injektionsnarkosemittel kommen entweder allein (TIVA) oder zusammen mit gasförmigen Narkosemitteln zum Einsatz. Die intravenös applizierbaren Narkosemittel, die z. Zt. eingesetzt werden, zeichnen sich zwar durch einen sofortigen Wirkungseintritt aus, zeigen aber regelmäßig ein Bündel von Nachteilen. Hervorzuheben ist, daß diese, wenn überhaupt, nur eine geringe schmerzhemmende Wirkung entfalten und schlecht steuerbar sind. So steht dem Vorteil einer psychischen Schonung des Patienten bei der Narkoseeinleitung, wobei der Patient momentan das Bewußtsein verliert und die Gesichtsmaske und das Excitationsstadium erspart bleiben, der Nachteil des erhöhten Narkoserisikos gegenüber. Dieses Risiko liegt vor allem darin, daß der Anästhesist auf das einmal injizierte Narkosemittel so gut wie keinen Einfluß mehr nehmen kann, so daß der Narkoseverlauf nur von den sich im Organismus abspielenden Vorgängen - Verteilung, enzymatischer Abbau und Inaktivierung sowie Ausscheidung über Leber und Niere - bestimmt wird. Weitere Nachteile der z. Zt. zur Anwendung kommenden Injektionsnarkosemittel liegen in schlecht einzuschätzenden Nebenwirkungen (beispielsweise Blutdruckabfall, Bradykardie, Rigidität, allergische Reaktion) und z. T. gravierenden Kontraindikationen. Da bekannte intravenöse Anästhetika häufig zusammen mit Analgetika und Muskelrelaxanzien gegeben werden, wird durch diese zusätzlich die Pharmakokinetik, vor allem die Halbwertzeit, erheblich verändert. Dadurch wird eine Steuerbarkeit insgesamt wesentlich erschwert.

Anästhesie besteht aus Hypnose, Analgesie und Amnesie. Es gibt aber keinen einzigen intravenös zu verabreichenden anästhetisch wirksamen Stoff, der wirksam und in sicherer Weise diese drei Komponenten der Anästhesie bewerkstelligen kann. Um dieses Ziel zu erreichen, setzt man Wirkstoffkombinationen ein. Die zur Zeit bekannten Wirkstoffe beeinträchtigen sich sowohl hinsichtlich der Pharmakodynamik als auch der Pharmakokinetik gegenseitig. Insbesondere werden Nebenwirkungen verstärkt, die bei der Anästhesie nicht nur unerwünscht sondern auch gefährlich sein können. Hierzu zählen insbesondere ausgeprägte Wirkungen auf das Herz und die Blutgefäße sowie auf die kardiovaskulären Kontrollmechanismen.

Aus der US-A-4 622 219 ist ein lokales Anästhetikum bekannt, das durch Injektion verabreicht werden kann. Dieses Injektionslokalanästhetikum enthält Mikrotröpfchen, die zum größten Teil aus verdampfbaren Anästhetika, wie beispielsweise Methoxyfluran aufgebaut sind. Die Wirksamkeit dieser Lösung ist aber ausschließlich lokal anästhesierend. Eine allgemeine Anästhesie oder die Narkose eines Patienten wird weder beschrieben noch erwogen. In diesem Zusammenhang ist hervor-zuheben, daß Methoxyfluran gegenüber gasförmigen Inhalations-narkotika wie Xenon eine etwa 440 mal größere Wirksamkeit hat (ausgedrückt als minimale alveoläre Narkotikumkonzentration bei 1 atm (MAC); MAC-Werte in Vol.-%: Xenon, 71; Methoxyfluran, 0,16).

Es besteht somit ein erhebliches Bedürfnis nach einem intravenös applizierbaren Narkosemittel mit hoher Wirksamkeit, das die genannten Nachteile nicht aufweist.

Gegenstand der Erfindung in einem umfassenden Sinne ist eine flüssige Präparation, die ein lipophiles Edelgas gelöst bzw. dispergiert enthält.

Im Gegensatz zu den im Zusammenhang mit Xenon beschriebenen physiologischen Begrenzungen der Anwendung der Edelgase als Inhalationsanästhetikum sind die anästhesiologischen Möglichkeiten bei der intravenösen Anwendung eines lipophilen Edelgases, wie beispielsweise Xenon, völlig anders. Diese ermöglichen bisher nicht erkannte Verbesserungen und vor allem Sicherheiten für den Patienten. Bereits Präparationen, die eine recht geringe Menge Xenon enthalten, führen zu einer deutlichen Anästhesie und Analgesie. Dies ist völlig überraschend. Weiterhin wurde festgestellt, daß Xenon die Herzmuskulatur nicht negativ beeinflußt. Darüberhinaus übt Xenon keine Wirkung auf das Reizleitungssystem am Herzen aus. Somit wird der Herzrhythmus als auch die Herzkontraktilität in keiner Weise durch das Edelgas negativ beeinflußt. Mit der erfindungsgemäßen Präparation erzielt man bei einem Patienten sowohl eine komplette Anästhesie als auch Analgesie, die eine weitere Supplementierung durch andere intravenös zu verabreichende Sedativa, Anästhetika oder Analgetika überflüssig macht. Es können darüberhinaus Dosierungen erzielt werden, bei denen eine zentrale Muskelrelaxation erreicht wird, so daß eine Supplementierung mit Muskelrelaxanzien ebenfalls überflüssig wird. Somit kann ein Patient bereits bei der Narkoseeinleitung durch die mono-intravenöse Narkose mit dem lipophilen Edelgas anästhesiert, analgesiert und muskelrelaxiert werden, so daß eine problemlose Intubation möglich ist.

Die Erfindung eröffnet darüberhinaus neue Wege bei der Supplementierung einer intravenösen Medikation, z.B. wenn zusätzlich eine Sedierung eines Patienten angestrebt wird. Dies umfaßt u.a. auch die Nierenersatztherapieformen wie Hämofiltration, Hämodiafiltration, Hämodialyse und extrakorporale Membranoxygenierung oder extrakorporale CO₂-Elimination und Herzlungenmaschine. Im Rahmen dieser therapeutischen Verfahren kann dann Xenon einem Patienten zugeführt werden. Die erfindungsgemäße Präparation kann dann zusätzlich infundiert werden.

Erfindungsgemäß werden flüssige Präparationen bereitgestellt, die aufgrund einer gewissen Lipophilie ein fettlösliches Gas, wie das erwähnte Xenon oder Krypton, leicht aufnehmen können.

Gegenstand der Erfindung ist somit eine flüssige Präparation in der Form einer Emulsion für die Anästhesie, die in anästhetisch wirksamer Konzentration ein lipophiles Edelgas enthält. Gegenstand der Erfindung sind aber auch solche Präparationen in der Form einer Emulsion, die ein lipophiles Edelgas in einer sedierend, analgetisch, muskelrelaxierend oder antiinflammatorisch wirksamen Konzentration enthalten.

Perfluorcarbone lassen sich u.a. intrapulmonal applizieren, so daß auf diesem Wege zusammen mit einer Xenonbeladung einerseits eine akute Lungenschädigung behandelt werden kann, andererseits ist aber auch eine Anästhesie, Sedation und/oder Analgesie aufgrund der pharmakologischen Wirkung von Xenon möglich. Die intrapulmonale Applikation von Perfluorcarbon zusammen mit Xenon zur partiellen Flüssigkeitsventilation und ergänzend zur Anästhesie oder auch Schmerzbekämpfung ist ein neuer Ansatz bei der Behandlung schwerer respiratorischer Krisen. Hierbei werden mit konventioneller Therapie nicht erreichbare, kollabierte, atelektatische Lungenbezirke, wiedereröffnet, wodurch diese Lungenbezirke wieder für einen Gasaustausch bereitgestellt werden.

Perfluorcarbone lassen sich auch intravenös applizieren, so daß eine erfindungsgemäße Präparation auf dieser Grundlage zur i.v. Narkose mittels Xenon eingesetzt werden kann. Perfluorcarbone haben aber auch eine Sauerstoffbeladungskapazität, so daß es sich anbietet, zugleich mit Sauerstoff beladene Perfluorcarbone intravenös zu applizieren. Auf diesem Wege kann nicht nur eine Narkose sondern eine Narkose und eine (ergänzende) Sauerstoffversorgung erreicht werden. Im Rahmen erschwerter Intubationen jeglicher Art wird dadurch eine für einen Patienten bisher nicht bekannte Sicherheit - Vermeidung von Hypoxie - erzielt.

Es ist allgemein bekannt, daß Perfluorcarbonverbindungen ein hohe Löslichkeit hinsichtlich einer Vielzahl von Gasen haben. Eine erfindungsgemäße Perfluorcarbonemulsion besteht beispielsweise aus bis zu 90 % (Gewicht/Volumen) Perflubron (C₈F₁₇) Zusätzlich sind hier Emulgatoren, beispielsweise Phospholipide aus Hühnereigelb erforderlich. Über diese Emulsionen, die sich erfindungsgemäß mit Xenon beladen lassen, wird beispielsweise bei J.A. Wahr et al. in Anesth. Analg. 1996, 82, 103-7, berichtet.

Geeignete Fluorocarbonemulsionen umfassen vorzugsweise 20 w/v % bis 125 w/v % einer hochfluorierten Kohlenwasserstoffverbindung, beispielsweise mehrfach fluorierte Bisalkylethene, cyclische Fluorkohlenstoffverbindungen wie Fluor-dekalin oder Perfluor-dekalin, fluoriertes Adamantan, perfluorierte Amine wie fluoriertes Tripropylamin und fluoriertes Tributylamin. Es können auch monobromierte Perfluorcarbone eingesetzt werden wie beispielsweise 1-Bromheptadecafluoroctan (C₈F₁₇Br), 1-Brompenta-decafluorheptan (C₇F₁₅Br) und 1-Bromtridecafluorhexan (C₆F₁₃Br). Darüberhinaus sind weitere Verbindungen brauchbar, wie unter anderem perfluoralkylierte Ether oder Polyether, z.B. (CF₃)₂ CFO(CF₂CF₂)₂ OCF(CF₃)₂, (CF₃)₂ CFO(CF₂CF₂)₃ OCF(CF₃), (CF₃)₂ CFO(CF₂CF₂)₂F, (CF₃)₂ CFO(CF₂CF₂)₃F und (C₆F₁₃)₂O.

Weiterhin sind chlorierte Derivate der zuvor erwähnten Perfluorcarbone einsetzbar.

Die Trägerkapazität der zuvor erwähnten Perfluorcarbonpräparation ist beträchtlich. Es wurden z.B. Xenonbeladungen von 1 bis 10 ml/ml mit einfachsten Mitteln erreicht. Beispielsweise können diese Präparationen mit Edelgas durch einfaches Durchleiten des Gases beladen werden.

Gegenstand der Erfindung sind weiterhin Fettemulsionen, die in der Lipidphase gelöst oder dispergiert das lipophile Edelgas enthalten.

Es hat sich gezeigt, daß man Xenon in einer beträchtlichen Menge in einer Fettemulsion anreichern kann. So kann bereits mit einfachsten Mitteln Xenon in Konzentrationen von 0,2 bis 10 ml (Konzentrationen beziehen sich auf Standardbedingungen, d.h. 20°C und Normaldruck) und darüber pro ml Fettemulsion gelöst bzw. dispergiert werden. Die Xenon-Konzentration hängt von einer Vielzahl von Faktoren ab, insbesondere der Konzentration des Fetts. In der Regel wird man die erfindungsgemäßen Präparationen bis an die Sättigungsgrenze mit Xenon "beladen". Es können aber auch sehr geringe Konzentrationen vorliegen, soweit beispielsweise bei intravenöser Applikation eine pharmakologische Wirksamkeit noch beobachtet werden kann. Bei einer 10 %igen Fettemulsion können ohne weiteres Xenon-Konzentrationen von 0,3 bis 2 ml Xenon pro ml Fettemulsion erreicht werden. Selbstverständlich sind auch höhere Werte, wie z.B. 3, 4, 5, 6 oder 7 ml Xenon pro ml Fettemulsion erreichbar. Diese Fettemulsionen sind zumindest in gasdicht verschlossenen Behältern hinreichend stabil, so daß das Xenon sich während üblicher Lagerungszeiträume nicht wieder als Gas freisetzt.

Im Stand der Technik werden in einer Vielzahl von Dokumenten Kontrastmittel insbesondere für Ultraschalluntersuchungen beschrieben, die ein Gas enthalten. Wesentlich für solche Kontrastmittel ist es, daß eine separate Phase ausgebildet wird, die durch kleinste Gasblasen (oder sogar gasgefüllte Ballons) gebildet wird (siehe u.a. WO-A-96/39197, US-A-5 088 499, US-A-5 334 381, WO 96/41647). Als Gas werden eine Vielzahl von Gasen vorgeschlagen, insbesondere Luft, Stickstoff, Kohlenstoffdioxid, Sauerstoff, aber auch Edelgase allgemein (i.e. Helium, Argon, Xenon und Neon). Lediglich die EP-B-0 357 163 beschreibt konkret, daß insbesondere xenon-haltige Mittel als Röntgenkontrastmittel eingesetzt werden können. Auch hier wird betont, daß die Injektionslösung Gasblasen enthalten muß. An keiner Stelle findet sich aber auch nur ein Hinweis darauf, daß Xenon eine analgetische oder anästhesierende Wirkung beim Einsatz als Kontrastmittel hat. Tatsächlich wäre eine solche Wirkung auch unerwünscht. Darüberhinaus ist die Gaskonzentration in Kontrastmitteln auch so gering, daß die Grenzkonzentration für eine pharmakologische Wirkung nicht erreicht wird. Kontrastmittel als solche werden deshalb in der vorliegenden Anmeldung auch nicht beansprucht.

Die Lipidphase der Präparation, die das Gas aufnimmt, d.h. lösen und/oder dispergieren kann, wird im wesentlichen durch sog. Fette gebildet, wobei es sich im wesentlichen um Ester von langkettigen und mittellangkettigen Fettsäuren handeln kann. Solche Fettsäuren, gesättigt oder ungesättigt, enthalten 8 bis 20 Kohlenstoffatome. Daneben können aber auch omega-3- oder omega-6-Fettsäuren eingesetzt werden, die bis zu 30 Kohlenstoffatome enthalten können. Als veresterte Fettsäuren bieten sich insbesondere pflanzliche Öle an, wie z. B. Baumwollsamenöl, Sojabohnenöl, Distelöl, Fischöl und dgl. Hauptbestandteil dieser natürlich vorkommenden Öle sind die Triglyceride der Fettsäuren. Von besonderer Bedeutung sind Präparationen, die als sog. Öl-in-Wasser-Emulsionen vorliegen. Dabei macht der Fettanteil der Emulsion üblicherweise 5 bis 30 Gew.-%, vorzugsweise 10 bis 20 Gew.-% aus. Neben dem Fett ist aber in der Regel ein Emulgator vorhanden, wobei sich Sojaphosphatide, Gelatine oder auch Eiphosphatid bewährt haben. Solche Emulsionen können hergestellt werden, indem das mit Wasser nicht mischbare Öl in Gegenwart des Emulgators, in der Regel ein oberflächenaktives Mittel, in Wasser emulgiert wird. Neben dem Wasser können auch andere polare Lösemittel, wie beispielsweise Ethanol, Glycerin (Propylenglykol, Hexylenglykol, Polyethylenglykol, Glykolmonoether, ein mit Wasser mischbarer Ester, etc.) vorhanden sein. Das Edelgas kann bereits in einer vorausgehenden Verfahrensstufe in die Lipidphase eingebracht worden sein. Im einfachsten Fall bietet sich aber an, die fertiggestellte Emulsion mit dem Xenon zu beladen. Dies kann bei unterschiedlichen Temperaturen erfolgen, beispielsweise bei Temperaturen von 1°C bis zu Raumtemperatur. Hierbei ist es zuweilen hilfreich, das Gefäß, in dem sich die Emulsion befindet, mit einem Druck von beispielsweise bis zu 8 Atmosphären oder darüber zu beaufschlagen.

Erfindungsgemäß lassen sich Fettemulsionen einsetzen, wie sie bei der intravenösen Ernährung zum Einsatz kommen. Diese Fettemulsionen bestehen im wesentlichen aus einer geeigneten Fettgrundlage (Sojabohnenöl oder Sonnenblumenkernöl) und einem gutverträglichen Emulgator (Phosphatide). Allgemein gebräuchliche Fettemulsionen sind Intralipid®, Intrafat®, Lipofundin®S und Liposyn®. Genauere Angaben zu diesen Fettemulsionen kann man G. Kleinberger und H. Pamperl, Infusionstherapie, 108-117 (1983) 3, entnehmen. Die Fettemulsionen enthalten im allgemeinen noch Zusätze, die die Osmolarität der wäßrigen Phase, die die in Form von Liposomen vorliegende Fettphase umgibt, Blut-isoton machen. Hierzu kann man Glycerin und/oder Xylit verwenden. Darüberhinaus ist es häufig sinnvoll, der Fettemulsion ein Antioxidationsmittel zuzugeben, um eine Oxidation der ungesättigten Fettsäuren zu verhindern. Hierfür eignet sich insbesondere Vitamin E (DL-Tocopherol).

Als Lipidphase besonders vorteilhaft, insbesondere bei einer Öl-in-Wasser-Emulsion, sind sog. Liposomen, die sich aus den oben erwähnten Triglyceriden aber auch allgemein aus sog. Phospholipidmolekülen bilden lassen. Diese Phospholipidmoleküle bestehen im allgemeinen aus einem wasserlöslichen Teil, der durch mindestens eine Phosphatgruppe gebildet wird, und einem Lipidteil, der sich von einer Fettsäure bzw. deren Ester ableitet.

In der US-A-5 334 381 wird im Detail erläutert, wie man Liposomen mit Gas beladen kann. Ganz allgemein gesprochen wird eine Vorrichtung mit den Liposomen gefüllt, d.h. mit einer Öl-in-Wasser-Emulsion, und dann wird die Vorrichtung mit dem Gas darin unter Druck gesetzt. Dabei kann die Temperatur bis auf 1°C abgesenkt werden. Unter Druck löst sich das Gas allmählich auf und gelangt in die Liposomen. Bei einer Entspannung des Drucks kann es dann zur Ausbildung von kleinen Gasblasen kommen, die aber jetzt von den Liposomen eingekapselt werden. Somit ist es praktisch möglich, beispielsweise Xenongas oder andere Gase unter hyperbaren Bedingungen in einer Fettemulsion zu halten. Auch solche Präparationen können erfindungsgemäß verwendet werden, solange es nicht zur Ausbildung einer separaten Gasphase außerhalb der Liposomen kommt und vorausgesetzt, daß die angestrebte pharmakologische Wirkung eintritt.

Die Lipide, die die Liposomen ausbilden, können von natürlicher oder synthetischer Herkunft sein. Solche Materialien sind beispielweise Cholesterol, Phosphatidylcholin, Phosphatidylethanolamin, Phosphatidylserin, Phosphatidylglycerin, Phosphatidylinositol, Sphingomyelin, Glycosphingolipide, Glucolipide, Glycolipide, usw. Die Oberfläche der Liposomen kann weiterhin mit einem Polymer modifiziert sein, beispielsweise mit Polyethylenglycol.

Die erfindungsgemäßen Präparationen weisen nun eine Vielzahl von Vorteilen auf. So konnte beobachtet werden, daß bei der Injektion mit einer erfindungsgemäßen Präparation eine praktisch sofortige Narkosewirkung auftrat, die sich aber anders als bei allen bekannten injizierbaren Narkosemitteln leicht steuern ließ. Das erfindungsgemäße Mittel wirkt aber nicht nur anästhesierend sondern zugleich schmerzstillend und beim Aufwachen euphorisierend. Die Elimination aus dem Körper ist ausschließlich abhängig von der Atmung. Darüberhinaus kann bei einem intravenös verabreichten Narkosemittel in der Exspirationsluft die Xenon-Konzentration leicht gemessen werden. Auf diesem Wege kann eine Narkosesteuerung erreicht werden, wie sie bislang mit üblichen intravenös applizierten Narkosemitteln nicht möglich war.

Gegenstand der Erfindung sind somit medizinische flüssige Präparationen in Form von Emulsionen, die ein lipophiles Edelgas in einer pharmakolo-gisch wirksamen Konzentration enthalten. Pharmakologisch wirksam heißt hier anästhesierend (auch subanästhesierend), analgesierend, muskelrelaxierend, sedierend und/oder antiinflammatorisch. Um eine beispielsweise subanästhesierende Wirkung zu erreichen, genügt bereits eine Xenonbeladung der medizinischen Präparation von etwa 0,2 bis 0,3 ml Xenon pro ml Emulsion. Das bedeutet, daß eine analgesierende und/oder sedierende Wirkung bei Präparationen mit einen Xenongehalt von mindestens 0,2 ml/ml Emulsion gewährleistet ist. Eine antiinflammatorische Wirkung wird schon bei 0,1 ml/ml Emulsion beobachtet. Es hat sich gezeigt, daß 20 ml einer Emulsion, die 0,3 ml Xe pro ml Emulsion enthält, bei kontinuierlicher Infusion über 30 sec einen subanästhesierten Zustand bei einem etwa 85 kg schweren Patienten ermöglicht. Wenn man mit einer hochbeladenen Perfluorcarbonemulsion arbeitet, die beispielsweise 2 bis 4 ml Xenon pro ml Emulsion enthält, so wird zur Narkoseeinleitung beispielsweise 20 ml dieser Emulsion über 30 sec infundiert. Zur Aufrechterhaltung der Anästhesie reicht eine Infusionsrate von mindestens 7,5 ml/min. Insgesamt würden somit 470 ml Emulsion für eine 1-stündige Operation anfallen. Das entspricht einem Xenonvolumen bei einem Xenongehalt von 3 ml Xenon pro ml Emulsion von 1410 ml, also einem Bruchteil des Xenonverbrauchs bei einer Inhalationsnarkose (bezogen auf ein Körpergewicht von 85 kg wäre das ein Verbrauch von 16,6 ml pro kg in einer Stunde).

Die erfindungsgemäße Präparation läßt sich nun mit jedem bekannten Inhalationsanästhetikum kombinieren, d.h. neben eine i.v. Applikation tritt eine Inhalationsanästhesie. In der kombinierten Verwendung mit Lachgas oder Xenon und/oder mit anderen Narkosemitteln, wie Halothan, Diethylethern, Sevofluran, Desfluran, Isofluran, Ethran etc. kann unter Umständen die Konzentration bzw. die Menge des eingesetzten Inhalationsnarkosemittels vermindert werden.

Darüberhinaus ist es möglich und unter Umständen auch vorteilhaft, in der Präparation neben dem Edelgas ein weiteres pharmakologisch wirksames Mittel vorzusehen. Dies kann beispielsweise ein intravenöses Sedativum oder Anästhetikum sein. Je nachdem ob es sich hierbei um ein wasserlösliches oder fettlösliches Mittel handelt, ist dieses dann in der wäßrigen Phase oder der Lipidphase neben dem Xenon vorhanden. Als besonders geeignet hierfür bietet sich 2,6-Diisopropylphenol an, wobei es sich um ein wirksames Anästhetikum handelt (beispielsweise 1,5 - 20 mg/ml). Geeignet ist auch Etomidat in Konzentrationen von 0,1 - 2 mg/ml (Hypnomidate®, ein Imidazol-5-carbonsäurederivat). Durch den Einsatz von gelöstem Xenon neben dem weiteren Anästhetikum läßt sich die zur Narkotisierung notwendige Konzentration von bsp. Diisopropylphenol oder Etomidat auf kleinere Werte absenken. So kann beispielsweise 1 ml erfindungsgemäße Fettemulsion (die etwa 0,1 g Fett pro ml Emulsion enthält) 2,5 - 20 mg 2,6-Diisopropylphenol enthalten, d. h. beispielsweise 2,5, 5,0, 7,5, 10, 15 oder 20 mg neben dem Xenon.

Es können ganz allgemein neben dem Xenon als anästhesierend, analgetisch oder sedierend wirkender Substanz weitere Anästhetika, Analgetika, Muskelrelaxanzien oder Sedativa vorhanden sein. Als weitere Anästhetika kommen z.B. Barbiturate (u.a. Barbital, Phenobarbital, Pentobarbital, Secobarbital, Hexobarbital und Thiopental) im allgemeinen und Opioide in Betracht. Bekannte Analgetika sind u.a. Verbindungen vom Typ des Morphins, z.B. Hydromorphon, Oximorphon, Codein, Hydrocodon, Thebacon, Thebain, Heroin. Weiterhin können synthetische Derivate des Morphins verwendet werden, z.B. Pethidin, Levomethadon, Dextromoramid, Pentazocin, Fentanyl, Alfentanil. Auch schwächer wirkende Analgetika können zum Einsatz kommen wie Anthranilsäurederivate (Flufenaminsäure, Mefenaminsäure), Acrylsäurederivate (Diclofenac, Tolmetin, Zomepirac), Arylpropylsäurederivate (Ibuprofen, Naproxen, Phenoprofen, Ketoprofen) und Indol- bzw. Indenessigsäurederivate (Indometacin, Sulindac). Als Muskelrelaxanzien können zentrale Muskelrelaxanzien eingesetzt werden wie beispielsweise Baclofen, Carisoprodol, Chlordiazepoxid, Chlormezanon, Chloroxazon, Dantrolen, Diazepam, Phenyramidol, Meprobamat, Phenprobamat, Orfenadrin). Sedativa, die erfindungsgemäß eingesetzt werden können, sind u.a. Benzodiazepinderivate wie Triazolam, Lormetazeban, Clotiazepam, Flurazepam, Nitrazepam, Flunitrazepam.

Mit einer erfindungsgemäßen Präparation lassen sich folglich mehrere Ziele realisieren:
a) intravenöse Narkoseeinleitung (ggf. unterstützt durch Anteile an 2,6-Diisopropylphenol oder Etomidat),
b) ergänzende intravenöse Applikation parallel zu einer Inhalationsanästhesie mit Xenon oder einem anderen Gas (z. B. Lachgas oder Desflurane) wobei die einzusetzende Gasmenge insgesamt beträchtlich vermindert werden kann,
c) Aufrechterhaltung der Narkose über längere Zeit, wobei das Edelgas-haltige Präparat ggf. nur ergänzend neben beispielsweise 2,6-Diisopropylphenol oder Etomidat verabreicht wird; da hier die Konzentration von beispielsweise Diisopropylphenol beträchtlich vermindert werden kann, wird so eine praktisch nebenwirkungsfreie Dauernarkose möglich,
d) durch die analgetische Wirkung des Xenons kann bei einer Kombination mit einem Inhalationsanästhetikum oder einem intravenösem Anästhetikum die zusätzliche Supplementierung mit einem Analgetikum erheblich reduziert oder vollständig vermieden werden,
e) intravenöse Xenonpräparate mit und ohne Kombination mit inhalativen oder intravenösen Anästhetika reduzieren den Bedarf an Muskelrelaxanzien bis hin zur völligen Vermeidung.

Wie sich aus den vorstehenden Ausführungen ergibt, beschränkt sich die Erfindung nicht auf das Einsatzgebiet der Anästhesie. Der Ausdruck Anästhesie umfaßt hier sowohl die Einleitung als auch die Aufrechterhaltung einer Anästhesie. Daneben haben die erfindungsgemäßen Präparationen aber auch eine schmerzausschaltende Wirkung, die neben einer Anästhesie bedeutsam werden kann. Unter Umständen tritt aber auch die Schmerzausschaltung in den Vordergrund, beispielsweise akute und chronische Schmerztherapie, wobei eine gewisse subanästhesierende oder sedierende zusätzliche Wirkung häufig erwünscht ist. Durch die intravenöse Verabreichung in subanästhesierender Dosierung über einen langen Zeitraum (1 h bis mehrere Tage) wird eine verstärkt schmerzhemmende Wirkung erreicht. Ein besonderes Einsatzgebiet einer erfindungsgemäßen Präparation als Narkosemittel ist die Katastrophenmedizin. Hier sind häufig ganz besonders kurze Aufwachphasen bei vorausgehender tiefer schmerzfreier Narkose gefordert. Ein weiteres Beispiel ist die Akutbehandlung eines Herzinfarktes. Die erfindungsgemäße Präparation dient dann der Senkung des Sympathikotonus und der Schmerzbekämpfung. Das erfindungsgemäße Mittel läßt sich also auch ganz allgemein bei der Inflammations- und Schmerztherapie einsetzen. Hierbei bietet sich u. a. auch an, das erfindungsgemäße Mittel topisch zu verwenden.

Es sind darüberhinaus Salben und Cremes (Fettemulsion oder Liposomen) und dgl. denkbar, die beispielsweise auf verletztes Gewebe aufgebracht werden können. Diese Präparationen können auch in Hohlräume oder Gelenke eingespritzt werden, um eine pharmakologische Wirkung zu erreichen.

Erfindungsgemäße Salben oder Cremes sind besonders geeignet zur lokalen Schmerzbekämpfung. Hierbei wird die Salbe auf die zu behandelnde Stelle aufgetragen, und ggf. mit einem luftdichten Wundverschluß versehen. Die Erfindung läßt sich folglich auch mittels eines Pflasters realisieren, das an der der Wunde zugewendeten Seite die erfindungsgemäße Präparation aufweist und an der von der Wunde fortweisenden Seite als übliche Pflasterauflage ausgebildet ist, die ggf. luftdicht ausgeführt ist.

Im weitesten Sinne läßt sich also die Erfindung verstehen als eine flüssige oder gelartige Präparation, die gelöst oder dispergiert das Edelgas enthält. Wie hier beispielhaft anhand einer Fettemulsion demonstriert, ist die erfindungsgemäße flüssige oder auch gelartige Präparation dadurch gekennzeichnet, daß sie in einer feinverteilten separaten Phase gelöst das Gas mit der pharmakologischen Wirkung enthält. Diese separate Phase ist in der Regel die disperse Phase einer Dispersion oder Emulsion. Die gashaltige separate Phase kann aber auch das Dispersionsmittel sein. Die erfindungsgemäßen Präparationen werden im allgemeinen so aufgebaut, daß die disperse Phase als solche die Eigenschaft hat das Gas zu lösen. Bei dem eingesetzten lipophilen Edelgas bietet sich deshalb eine Fettemulsion an mit separaten kleinsten Fetttröpfchen oder Liposomen, die dann das Edelgas gelöst enthalten. Ganz allgemein ist aber festzuhalten, daß die erfindungsgemäßen Präparationen vorzugsweise Emulsionen sind, bei denen in der Regel die disperse Phase das wirksame Gas enthält.

### Experimenteller Teil

### Fettemulsionen

In den folgenden Beispielen wurden als Fettemulsionen die käuflich erhältlichen Intralipidpräparate (erhältlich von Pharmacia & Upjohn GmbH, Erlangen) verwendet. Diese Emulsionen bestehen im wesentlichen aus Sojabohnenöl, 3-sn-Phosphatidylcholin (aus Hühnereigelb) und Glycerol. Eine Intralipid®10 Fettemulsion hat beispielsweise die folgende Zusammensetzung:

| | |
|---|---|
| Sojabohnenöl | 100 g |
| (3-sn-Phosphatidyl)cholin aus Hühnereigelb | 6 g |
| Glycerol | 22,0 g |
| Wasser für Injektionszwecke ad | 1000 ml |

Mit Natriumhydroxid auf pH 8,0 eingestellt.
Energiewert/l: 4600 kJ (1100 kcal)
Osmolarität: 260 mOsm/l

Eine Intralipid®20 Fettemulsion hat beispielsweise die folgende Zusammensetzung:

| | |
|---|---|
| Sojabohnenöl | 200 g |
| (3-sn-Phosphatidyl)cholin aus Hühnereigelb | 12 g |
| Glycerol | 22,0 g |
| Wasser für Injektionszwecke ad | 1000 ml |

Mit Natriumhydroxid auf pH 8,0 eingestellt.
Energiewert/l: 8400 kJ (2000 kcal)
Osmolarität: 270 mOsm/l

### Beladung von Perfluorcarbonemulsionen mit Xenon

Es wurde eine Reihe von Perfluorcarbonemulsionen hergestellt bzw. käuflich erworben und mit Xenon beladen. Die Wirksamkeit der Präparationen wurde am Tiermodell verifiziert (Kaninchen). Alle Emulsionen verhielten sich so wie die zuvor beschrieben Intralipidpräparate, d.h. das Versuchstier wurde durch eine Injektion in das Ohr kurzzeitig narkotisiert (etwa 1 ml).

Die Emulsionen wurden jeweils in einem Becherglas vorgelegt und durch Durchleiten des Xenongases beladen.

Folgende Perfluorcarbonverbindungen wurden eingesetzt:
Perfluorhexyloctan (1), Perfluordecalin (2), Perflubron (C₈F₁₇) (3).

Darüberhinaus wurden zur Herstellung der Emulsionen Emulgatoren eingesetzt, beispielsweise Eidotterlecithin (Lipoid E100 von der Lipoid GmbH, Ludwigshafen), Pluronic PE6800 und Pluronic F68.

Es wurde bei allen Emulsionen festgestellt, daß bereits eine 40 %ige Perfluorcarbonemulsion (Gewicht/Volumen; d.h. Gewicht Perfluorcarbonverbindung zu Volumen Emulsion) 1 bis 4 ml Xenon pro ml Emulsion aufnehmen konnte.

### Tierexperimentelle Studien

Um die Wirksamkeit der erfindungsgemäße Präparationen zu zeigen, wurde eine Untersuchung mit 24 Schweinen, Alter 14 bis 16 Wochen, mit einem Gewicht von 36,4 -43,6 kg durchgeführt. Insgesamt wurden zufallsbestimmt 6 Gruppen gebildet, die entweder in konventioneller Weise oder mittels der erfindungsgemäßen Emulsion anästhesiert wurde. In allen Gruppen wurde die Anästhesie mit einer Bolusinjektion Pentobarbiton (8 mg/kg Körpergewicht) und Buprenorphin (0,01 mg/kg Körpergewicht) intravenös eingeleitet. Dann wurde die Anästhesie fortgesetzt mittels konventioneller Inhalationsnarkotika (Lachgas oder Xenon-Sauerstoff-Mischung) oder einer intravenösen Applikation von Pentobarbiton und Buprenorphin. Bei einer Gruppe (Vergleichsgruppe) wurde die Anästhesie durch intravenöse Applikation von 2,6-Diisopropylphenol (10 mg/1 ml Emulsion) aufrechterhalten. Zwei Gruppen (erfindungsgemäß) Schweine zu je vier Individuen erhielten zur Aufrechterhaltung der Anästhesie eine Infusion intravenös von jeweils 1 ml/kg/h einer 10 gew.-%igen erfindungsgemäßen Fettemulsion, die mit Xenon zuvor gesättigt wurde (etwa 0,3 ml Xenon pro ml Emulsion). In der Gruppe 2 wurde zusätzlich 7,5 mg/kg Körpergewicht/h 2,6-Diisopropylphenol mit der Fettemulsion appliziert.

Die Schweine wurden einem chirurgischen Eingriff (Standardeingriff: Auftrennung der linken femoralen Arterie) unterzogen (identisch in jeder Gruppe und bei jedem Versuchstier) und es wurde der Adrenalinspiegel, die Herzfrequenz, der Arterienblutdruck und der Sauerstoffverbrauch aufgezeichnet. Weiterhin wurde festgehalten, welche zusätzlichen Pentobarbitongaben erforderlich waren, um die Analgesie und Narkosetiefe in jeder Gruppe auf das erforderliche Level zu bringen.

**Tabelle**

| Gruppe | Adrenalin pg/ml | Herzfrequenz [min^{-1]} | Arterienblutdruck [mm Hg] | VO₂ [ml/min] | Pentobarbitonerfordernis mg/kg/min |
|---|---|---|---|---|---|
| Vergleichsgruppe | 60 | 115 | 110 | 410 | 0,25 |
| | 134 | 120 | 105 | 391 | 0,36 |
| | 112 | 105 | 115 | 427 | 0,31 |
| | 85 | 98 | 101 | 386 | 0,42 |
| | | | | | |
| Gruppe 1 | 38 | 112 | 112 | 341 | 0,09 |
| | 21 | 106 | 100 | 367 | 0,04 |
| | 16 | 95 | 104 | 348 | 0,11 |
| | 30 | 112 | 118 | 334 | 0,15 |
| | | | | | |
| Gruppe 2 | 10 | 88 | 100 | 325 | - |
| | 23 | 100 | 85 | 346 | - |
| | 14 | 94 | 93 | 331 | - |
| | 8 | 104 | 87 | 354 | - |

Die Auswertung der Ergebnisse bezüglich der Gruppen, die konventionell narkotisiert wurden (hier nicht tabellarisch angegeben), zeigte, daß die Narkose mit einem Xenon-Sauerstoff-Gemisch den anderen Verfahren deutlich überlegen war. Überraschenderweise zeigten die zwei Gruppen, die eine erfindungsgemäße Fettemulsion (Gruppe 1 und Gruppe 2) intravenös appliziert bekommen hatten, ähnlich günstige Befunde, wobei bei einer kombinierten Applikation zusammen mit 2,6-Diisopropylphenol (Gruppe 2) sogar signifikante Verbesserungen erreicht werden konnten, was sich in einem geringeren Adrenalinspiegel (weniger Streß) und überhaupt keinem Pentobarbitonerfordernis zeigt.

Die in der Tabelle angegebenen Werte zeigen, daß die erfindungsgemäße Präparation allen z. Zt. erhältlichen intravenösen Narkosemitteln insbesondere wegen der zusätzlichen analgetischen Potenz überlegen ist. So zeigen die Schweine in der Gruppe 1 (10 gew.%ige Fettemulsion, gesättigt mit Xenon) im Vergleich (siehe Vergleichsgruppe) deutlich weniger Streß (Adrenalinspiegel), geringeren Sauerstoffbedarf (VO₂) und ein geringeres Pentobarbitonerfordernis (also eine bessere Narkose). Der Unterschied zu intravenös zu applizierenden Narkosemitteln nach dem Stand der Technik zeigt sich noch deutlicher, wenn man die Ergebnisse in der Gruppe 2 (10 %ige Fettemulsion mit 2,6-Diisopropylphenol und angereichert mit Xenon) der Vergleichsgruppe gegenüberstellt. Hier zeigt sich nicht nur ein deutlich verminderter Streß (Adrenalinspiegel). Bei deutlich beruhigter Herzfrequenz und geringerem Arterienblutdruck sowie geringerem Sauerstoffbedarf konnte auf zusätzliche Pentobarbitongaben verzichtet werden.

Bei einer weiteren Gruppe (4 Schweine a 31,4 bis 39,8 kg Körpergewicht) wurde der Einsatz von Perfluorcarbonpräparationen untersucht. Bei dieser Versuchsgruppe wurde eine 40 %ige Perfluorcarbonemulsion mit einem Xenongehalt von 2,1 ml Xenon pro ml Emulsion eingesetzt. Zur Einleitung und Intubation erhielten die Schweine intravenös 20 ml der Emulsion über 20 sec (das entspricht 1,34 ml Xenon/kg Körpergewicht). Nach der Intubation und Beatmung wurde Xenon intravenös kontinuierlich über 30 min infundiert. Dabei erhielten die Versuchstiere insgesamt 75 ml Emulsion (das entspricht 10 ml Xenon kg⁻¹h⁻¹).

In der folgenden Tabelle sind die Meßergebnisse für den Adrenalinspiegel, die Herzfrequenz, der Arterienblutdruck und den Sauerstoffverbrauch angegeben. Die Ergebnisse zeigen, daß bei höherer Xenonbeladung und größeren Infusionsraten (über 5 ml/kg/h) eine vollständige Narkose allein mit dem erfindungsgemäßen Mittel durchgeführt werden kann. Hierbei wird insgesamt sogar noch ein geringerer Sauerstoffbedarf (V̇O₂) und eine streßfreiere Narkose (Adrenalinspiegel und Herzfrequenz) festgestellt.

| Adrenalin [pg/ml] | Herzfrequenz [min⁻¹] | Arterienblutdruck [mm Hg] | V̇O₂ [ml/min] |
|---|---|---|---|
| 8 | 90 | 101 | 301 |
| 6 | 87 | 96 | 320 |
| 10 | 94 | 98 | 308 |
| 5 | 100 | 106 | 316 |

## Patentansprüche

1. Flüssige Präparation in der Form einer Emulsion für die Anästhesie, die in anästhetisch wirksamer Konzentration ein lipophiles Edelgas enthält.

2. Flüssige Präparation in der Form einer Emulsion für die Sedierung, die in einer sedierend wirksamen Konzentration ein lipophiles Edelgas enthält.

3. Flüssige Präparation in der Form einer Emulsion für die Analgesie, die in einer analgetisch wirksamen Konzentration ein lipophiles Edelgas enthält.

4. Flüssige Präparation in der Form einer Emulsion für die Muskelrelaxierung, die ein lipophiles Edelgas in einer muskelrelaxierend wirksamen Konzentration enthält.

5. Flüssige Präparation in der Form einer Emulsion für die Inflammationstherapie, die ein lipophiles Edelgas in einer antiinflammatorisch wirksamen Konzentration enthält.

6. Flüssige Präparation nach einem der vorstehenden Ansprüche, die gelöst oder dispergiert Xenon enthält.

7. Präparation nach einem der vorstehenden Ansprüche, die 0,2 bis 10 ml Xenon pro ml Emulsion enthält.

8. Präparation nach Anspruch 7, die 0,3 bis 2 ml Xenon pro ml Emulsion enthält.

9. Präparation nach Anspruch 7, die 1 bis 5 ml Xenon pro ml Emulsion enthält.

10. Präparation nach einem der vorstehenden Ansprüche, wobei die Präparation als Perfluorcarbonemulsion vorliegt.

11. Präparation nach einem der Ansprüche 1 bis 9, wobei die Präparation als Fettemulsion vorliegt.

12. Präparation nach Anspruch 11, die eine Öl-in-Wasser-Emulsion oder -Dispersion ist.

13. Präparation nach Anspruch 11 oder 12, mit einer Lipidphase, die im wesentlichen durch Liposomen gebildet wird.

14. Präparation nach einem der vorstehenden Ansprüche, wobei zusätzlich ein weiteres pharmakologisches Mittel in gelöster Form vorliegt.

15. Präparation nach Anspruch 14, wobei das zusätzliche pharmakologisch wirksame Mittel ein Anästhetikum, Analgetikum, Sedativum oder Muskelrelaxanz zur intravenösen Verabreichung ist.

16. Präparation nach Anspruch 15, wobei das zusätzliche pharmakologisch wirksame Mittel 2,6-Diisopropylphenol oder Etomidat oder ein Derivat davon ist.

17. Präparation nach Anspruch 15, wobei das zusätzliche pharmakologisch wirksame Mittel Fentanyl oder Alfentanil ist.

18. Infusionsmittel für die Anästhesie, das eine Präparation nach einem der vorstehenden Ansprüche umfaßt.

19. Verwendung einer xenonhaltigen flüssigen Emulsion zur Einleitung und/oder Aufrechterhaltung der Anästhesie.

## Claims

1. Liquid preparation in the form of an emulsion for anaesthesia, which contains a lipophilic noble gas in a concentration effective as an anaesthetic.

2. Liquid preparation in the form of an emulsion for sedation, which contains a lipophilic noble gas in a concentration effective as a sedative.

3. Liquid preparation in the form of an emulsion for analgesia, which contains a lipophilic noble gas in a concentration effective as an analgesic.

4. Liquid preparation in the form of an emulsion for muscular relaxation, which contains a lipophilic noble gas in a concentration effective as a muscle relaxant.

5. Liquid preparation in the form of an emulsion for inflammation therapy, which contains a lipophilic noble gas in a concentration effective as an anti-inflammatory.

6. Liquid preparation according to any one of the preceding claims which contains xenon in dissolved or dispersed form.

7. Preparation according to any one of the preceding claims which contains 0.2 to 10 ml of Xenon per ml of emulsion.

8. Preparation according to Claim 7, which contains 0.3 to 2 ml of Xenon per ml of emulsion.

9. Preparation according to Claim 7, which contains 1 to-5 ml of Xenon per ml of emulsion.

10. Preparation according to any one of the preceding claims, which is in the form of a perfluorocarbon emulsion.

11. Preparation according to any one of Claims 1 to 9 which is in the form of a fatty emulsion.

12. Preparation according to Claim 11, which is an oil-in-water emulsion of dispersion.

13. Preparation according to Claim 11 or 12 with a lipid phase which is formed essentially of liposomes.

14. Preparation according to any one of the preceding claims in which another pharmacological agent is additionally present in dissolved form.

15. Preparation according to Claim 14, wherein the additional pharmacologically active agent is an intravenous anaesthetic, analgesic, sedative or muscle relaxant.

16. Preparation according to Claim 15, wherein the additional pharmacologically active agent is 2,6-diisopropylphenol, etomidate or a derivative thereof.

17. Preparation according to Claim 15, wherein the additional pharmacologically active agent is fentanyl or alfentanil.

18. Infusion agent for anaesthesia, which comprises a preparation according to any one of the preceding claims.

19. Use of a Xenon-containing liquid emulsion for the induction and/or maintenance of anaesthesia.

## Revendications

1. Préparation liquide sous la forme d'une émulsion pour l'anesthésie, qui contient en concentration efficace pour l'anesthésie un gaz rare lipophile.

2. Préparation liquide sous la forme d'une émulsion pour la sédation, qui contient en concentration efficace pour la sédation un gaz rare lipophile.

3. Préparation liquide sous la forme d'une émulsion pour l'analgésie, qui contient en concentration efficace pour l'analgésie un gaz rare lipophile.

4. Préparation liquide sous la forme d'une émulsion pour la relaxation musculaire, qui contient un gaz rare lipophile en concentration efficace pour la relaxation musculaire.

5. Préparation liquide sous la forme d'une émulsion pour le traitement d'inflammations, qui contient un gaz rare lipophile en concentration efficace contre les inflamations.

6. Préparation liquide selon l'une des revendications précédentes, qui contient du xénon dissous ou dispersé.

7. Préparation selon l'une des revendications précédentes, qui contient de 0,2 à 10 ml de xénon par ml d'émulsion.

8. Préparation selon la revendication 7, qui contient de 0,3 à 2 ml de xénon par ml d'émulsion.

9. Préparation selon la revendication 7, qui contient de 1 à 5 ml de xénon par ml d'émulsion.

10. Préparation selon l'une des revendications précédentes, dans laquelle la préparation est présente sous la forme d'une émulsion de perfluorocarbone.

11. Préparation selon l'une des revendications 1 à 9, dans laquelle la préparation est présente sous la forme d'une émulsion grasse.

12. Préparation selon la revendication 11, qui est une émulsion ou une dispersion huile-dans-eau.

13. Préparation selon la revendication 11 ou 12, avec une phase lipidique, qui, pour l'essentiel, est constituée par des liposomes.

14. Préparation selon l'une des revendications précédentes, dans laquelle, en outre, un autre produit pharmacologique est présent sous forme dissoute.

15. Préparation selon la revendication 14, dans laquelle le produit actif pharmacologique supplémentaire est un anesthésique, un analgésique, un sédatif ou un relaxant musculaire pour une administration intraveineuse.

16. Préparation selon la revendication 15, dans laquelle le produit actif pharmacologique supplémentaire est le 2,6-diisopropylphénol ou l'étomidat, ou un dérivé de ceux-ci.

17. Préparation selon la revendication 15, dans laquelle le produit actif pharmacologique supplémentaire est le fentanyl ou l'alfentanil.

18. Produit de perfusion pour l'anesthésie, qui comprend une préparation selon l'une des revendications précédentes.

19. Utilisation d'une émulsion liquide contenant du xénon pour l'obtention et/ou la poursuite de l'anesthésie.
